(19) Europäisches Patentamt
European Patent Office
Office européen des brevets

(11) Veröffentlichungsnummer: **0 111 844**
**B1**

(12) # EUROPÄISCHE PATENTSCHRIFT

(45) Veröffentlichungstag der Patentschrift: **27.11.85**

(51) Int. Cl.⁴: **C 07 C 103/30,** C 07 C 103/44, C 07 C 103/82, C 07 C 102/04

(21) Anmeldenummer: **83112386.4**

(22) Anmeldetag: **09.12.83**

(54) **Verfahren zur Herstellung von 3-Acylaminoanilinen.**

(30) Priorität: **15.12.82 DE 3246367**

(43) Veröffentlichungstag der Anmeldung: **27.06.84 Patentblatt 84/26**

(45) Bekanntmachung des Hinweises auf die Patenterteilung: **27.11.85 Patentblatt 85/48**

(84) Benannte Vertragsstaaten: **CH DE FR GB IT LI**

(56) *Entgegenhaltungen:* **DE - A - 2 455 212**

(73) Patentinhaber: **HOECHST AKTIENGESELLSCHAFT, Postfach 80 03 20, D-6230 Frankfurt am Main 80 (DE)**

(72) Erfinder: **Hähnle, Reinhard, Dr., Kastanienweg 7a, D-6240 Königstein/Taunus (DE)**
Erfinder: **Grewer, Theodor, Dr., Humperdinckweg 18, D-6232 Bad Soden am Taunus (DE)**

**EP 0 111 844 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Es ist bekannt, 3-Acylaminoaniline durch partielle Acylierung von 1,3-Diaminobenzolen herstellen zu können. So wird beispielsweise in der European Patent Specification No. 0011048 ein Verfahren zur Herstellung von Verbindungen der allgemeinen Formel

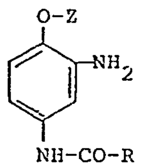

in welcher R und Z jeweils eine Alkylgruppe bedeuten, durch partielle Acylierung der entsprechenden 1,3-Diaminobenzolverbindungen beschrieben. Dieses Verfahren versagt aber im Fall von unsubstituierten 3-Acylaminoanilinen, weil die Möglichkeit der partiellen Acylierung gemäss dem bekannten Verfahren auf der deutlich verschiedenen Reaktivität der in den 2,4-Diaminophenoläthern enthaltenen beiden Aminogruppen beruht.

Das in der DE-OS 2455212 beschriebene Verfahren zur Monoacylierung eines sulfonsäuregruppenfreien primären Diamins beruht darauf, dass in der salzsauren Lösung des 1,3-Diaminobenzols die acylierbare, monoprotonierte Verbindung der Formel

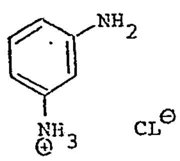

noch in ausreichender Konzentration vorliegt, weil die vorhandene positive Ladung die Zweitprotonierung erschwert, während die Monoacylverbindung praktisch vollständig als Hydrochlorid der Formel

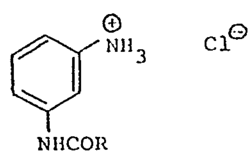

in welcher R eine Alkylgruppe bedeutet, vorliegt, welches nicht zur Diacylverbindung acyliert wird.

Bei den genannten bekannten Verfahren werden zur Acylierung Carbonsäurechloride oder -anhydride eingesetzt, und zwar im Überschuss, und die erhaltenen Reaktionsprodukte werden durch Aussalzen und Abfiltrieren isoliert, wobei ein stark belastetes Abwasser anfällt. Die 3-Acylaminoaniline werden bei diesen Verfahren als feuchte Hydrochloride erhalten, aus welchen die freien Basen durch Neutralisation erhalten werden können, wobei wieder Abwasser anfällt.

Es wurde nun gefunden, dass man 3-Acylaminoaniline der allgemeinen Formel (1)

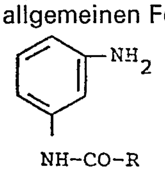

(1)

in welcher R einen verzweigten oder unverzweigten Alkylrest von 1-7 Kohlenstoffatomen oder die Phenylgruppe, die durch 1-2 Methylgruppen oder durch 1-2 Chloratome substituiert sein kann, bedeutet, abwasserfrei und in guten bis sehr guten Ausbeuten herstellen kann, indem man eine Carbonsäure der allgemeinen Formel (2)

$$R{-}COOH \qquad (2)$$

in welcher R die genannte Bedeutung hat, mit einem Überschuss an 1,3-Diaminobenzol umsetzt und die Reaktionsmischung durch Destillation trennt. Zweckmässigerweise lässt man das bei der Reaktion entstehende Wasser während der Reaktion abdestillieren.

Als Carbonsäuren kommen gesättigte Fettsäuren von 2 bis 8 Kohlenstoffatomen in Frage sowie Benzoesäure und deren im Benzolkern durch 1 oder 2 Methylgruppen oder 1 oder 2 Chloratome substituierten Derivate. In Betracht kommen insbesondere die Essigsäure, Propionsäure, Buttersäure, Isobuttersäure, Valeriansäure und Benzoesäure.

Das 1,3-Diaminobenzol wird in einem zwei- bis zehnfachen, vorzugsweise drei- bis vierfachen molaren Überschuss gegenüber der Carbonsäure eingesetzt.

Die Umsetzung erfolgt bei 120-240°C, vorzugsweise 155-210°C.

Es ist zweckmässig, die destillative Aufarbeitung des anfallenden Reaktionsgemisches bei vermindertem Druck von 13,3 bis 6650 Pa (0,1 bis 50 Torr) durchzuführen.

Das Verfahren beruht darauf, dass die Diacylierung durch den Überschuss an 1,3-Diaminobenzol statistisch zurückgedrängt wird und darauf, dass bei der destillativen Aufarbeitung keine unerwünschte Nebenreaktion, zum Beispiel keine Umacylierung, erfolgt, das heisst, dass die Reaktion

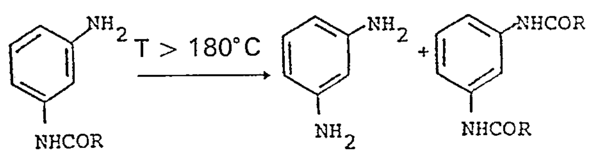


nicht stattfindet, was in Anbetracht der hohen thermischen Belastung bei der Destillation als überraschend zu erachten ist.

Unter der Annahme, dass jede im Reaktionsgemisch vorhandene Aminogruppe mit derselber Wahrscheinlichkeit reagiert, erhält man für die Zusammensetzung des Reaktionsgemisches folgende Gleichungen:

$$A = Ao - B + \frac{B^2}{4\,Ao}; \quad C = B - \frac{B^2}{2\,Ao}; \quad D = \frac{B^2}{4\,Ao}$$

In diesen Gleichungen bedeutet

Ao = Anzahl der eingesetzten Mole Diamin;

A = Anzahl der Mole Diamin im Reaktionsgemisch;

B = Anzahl der Mole Carbonsäure, die reagiert haben;

C = Anzahl der Mole Monoacylverbindung im Reaktionsgemisch;

D = Anzahl der Mole Diacylverbindung im Reaktionsgemisch.

Es zeigte sich jedoch, dass eine Aminogruppe im 1,3-Diaminobenzol um den Faktor 1,19 schneller reagiert als die Aminogruppe im 3-Acylaminoanilin, was als überraschend anzusehen ist.

Gegenüber dem in der weiter oben genannten DE-OS 2455212 beschriebenen Verfahren hat das erfindungsgemässe Verfahren folgende Vorteile:

1. Es entsteht kein Abwasser.

2. Die 3-Acylaminoaniline fallen nicht als Hydrochloride an, sondern als freie, trockene basen, was für die Weiterverarbeitung zu Handelsfarbstoffen vorteilhaft ist.

3. Es werden keine Säurechloride bzw. Säureanhydride gebraucht, sondern man kann die Säuren selbst einsetzen.

In Anbetracht der strengen Umweltschutzbestimmungen überwiegen die genannten Vorteile den Nachteil des höheren Energieeinsatzes.

Die verfahrensgemäss erhältlichen 3-Acylaminoaniline der genannten allgemeinen Formel (1) stellen wichtige Zwischenprodukte zur Herstellung von Azofarbstoffen dar.

*Beispiel 1:*

In einem Rührkolben, versehen mit einer 30 cm langen Kolonne, Kühler und Vorlage werden 756 g geschmolzenes 1,3-Diaminobenzol (7,0 Mol) und 222 g (3,0 Mol) Propionsäure vorgelegt. Innerhalb einer Stunde steigert man gleichmässig die Temperatur auf 170°C im Kolbeninneren, wobei Wasser abdestilliert. Dann hält man 1 h bei 170-180°C. Anschliessend wird das Reaktionsgemisch destillativ in drei Fraktionen zerlegt:

1. *Fraktion* (bis 100°C/133 Pa (1 Torr)) 72,5 g

2. *Fraktion* (bis 160°C/133 Pa (1 Torr)) 517 g 1,3-Diaminobenzol

3. *Fraktion* Rückstand, 362,7 g 3-Propionylaminoanilin und 1,3-Dipropionylaminobenzol.

Der Rückstand muss frei von 1,3-Diaminobenzol sein, was der Fall ist, wenn am Kopfthermometer 3-propionylaminoanilin erscheint, wovon man sich chromatographisch überzeugt.

Die 1. Fraktion wird mit 1n-Natronlauge titriert, dann angesäuert und mit 1n-Natriumnitritlösung titriert.

Die 2. Fraktion wird gewogen, der Rückstand wird gewogen und mit Natriumnitritlösung titriert.

Ergebnis:

1. Fraktion: 0,532 Mol Propionsäure und 0,04 Mol 1,3-Diaminobenzol.

2. Fraktion: 4,787 Mol 1,3-Diaminobenzol.

3. Fraktion: 1,99 Mol 3-Propionylaminoanilin und 0,165 Mol 1,3-Dipropionylaminobenzol.

Vom eingesetzten 1,3-Diaminobenzol wurden 6,98 Mol wiedergefunden.

Von der eingesetzten Propionsäure wurden 2,85 Mol wiedergefunden.

Die Ausbeute an 3-Propionylaminoanilin, bezogen auf verbrauchtes 1,3-Diaminobenzol, beträgt 91,5% der Theorie.

Das erhaltene 3-Propionylaminoanilin wurde zu dem roten Handelsfarbstoff der Formel

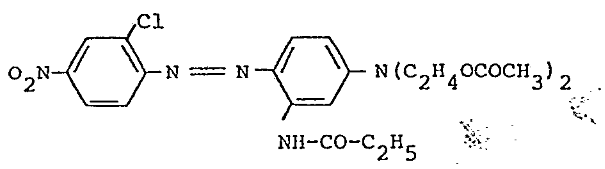

verarbeitet, welcher mängelfrei erhalten wurde.

*Beispiel 2:*

In einem Rührkolben, versehen mit einer 30 cm langen Kolonne, Kühler und Vorlage werden 756 g 1,3-Diaminobenzol (7,0 Mol) und 120 g Essigsäure (2,0 Mol) vorgelegt. Man verfährt weiter, wie in Beispiel 1 beschrieben. Die Aufarbeitung durch Destillation ergab:

1. *Fraktion* (100°C/133 Pa (1 Torr)) 50,5 g (0,395 Mol) Essigsäure enthaltend.

2. *Fraktion* (bis 160°C/133 Pa (1 Torr)) 579 g (5,36 Mol) 1,3-Diaminobenzol enthaltend.

3. *Fraktion* (Rückstand) 238,5 g, davon 1,56 Mol (234 g) 3-Acetylaminoanilin und 0,023 Mol (4,5 g) 1,3-Diacetylaminobenzol.

Die Ausbeute, bezogen auf verbrauchtes 1,3-Diaminobenzol, beträgt 95,1% der Theorie.

*Beispiel 3:*

In einem Rührkolben, versehen mit einer 30 cm langen Kolonne, Kühler und Vorlage werden 756 g 1,3-Diaminobenzol (7,0 Mol) und 264 g Buttersäure (3,0 Mol) vorgelegt. Dann verfährt man wie in Beispiel 1 beschrieben. Die Aufarbeitung durch Destillation ergab:

1. *Fraktion* (bis 100°C/133 Pa (1 Torr)) 108 g (0,719 Mol) Buttersäure und 0,05 Mol 1,3-Diaminobenzol enthaltend.

2. *Fraktion* (bis 160°C/133 Pa (1 Torr)) 524 g 1,3-Diaminobenzol (4,852 Mol)

3. *Fraktion* (Rückstand) 379 g, enthaltend 1,954 Mol 3-Butyraminoanilin (347,8 g) und 31,2 g 1,3-Dibutyraminobenzol (0,125 Mol)

Die Ausbeute, bezogen auf verbrauchtes 1,3-Diaminobenzol, beträgt 93% der Theorie.

*Beispiel 4:*

Verwendet man statt Buttersäure Isobuttersäure und verfährt im übrigen wie in Beispiel 3 beschrieben, so erhält man 1,465 Mol 3-Isobutyranilin, was, bezogen auf das verbrauchte 1,3-Diaminobenzol, einer Ausbeute von 92% der Theorie entspricht.

*Beispiel 5:*

In der in Beispiel 1 beschriebenen Apparatur werden 756 g 1,3-Diaminobenzol (7,0 Mol) und 306 g n-Valeriansäure vorgelegt. Anschliessend verfährt man wie in Beispiel 1 beschrieben, wobei man erhält:

1. *Fraktion*: 164 g, enthaltend 0,672 Mol Valeriansäure und 0,37 Mol 1,3-Diaminobenzol

2. *Fraktion*: 4,40 Mol 1,3-Diaminobenzol

3. *Fraktion*: (Rückstand) 417 g, enthaltend 2,00 Mol 3-Valerylaminoanilin und 0,12 Mol 1,3-Divalerylaminobenzol.

Die Ausbeute, bezogen auf verbrauchtes 1,3-Diaminobenzol, beträgt 89,7% der Theorie.

*Beispiel 6:*

In der in Beispiel 1 beschriebenen Apparatur werden 756 g 1,3-Diaminobenzol (7,0 Mol) und 148 g (2,0 Mol) Propionsäure vorgelegt. Anschliessend heizt man rasch auf 120° C und steigert dann die Temperatur im Verlauf von 5 h auf 200° C. Man führt die allmähliche Temperatursteigerung so durch, dass der über die Kolonne abgehende Dampf am Kolonnenkopf die Temperatur von 102° C nicht überschreitet. Anschliessend arbeitet man wie in Beispiel 1 beschrieben auf:

1. *Fraktion*: 48 g, enthaltend 0,13 Mol Propionsäure und 0,051 Mol 1,3-Diaminobenzol.

2. *Fraktion*: 553 g (5,12 Mol) 1,3-Diaminobenzol.

3. *Fraktion*: 298,5 g, enthaltend 1,712 Mol 3-Propionylaminoanilin (280,8 g) und 17,7 g 1,3-Dipropionylaminobenzol.

Die Ausbeute an 3-Propionylaminoanilin, bezogen auf verbrauchtes 1,3-Diaminobenzol, beträgt 93,6% der Theorie.

*Beispiel 7:*

In der in Beispiel 1 beschriebenen Apparatur werden 756 g 1,3-Diaminobenzol (7,0 Mol) und 244 g Benzoesäure (2,0 Mol) 4 h auf 200-210° C erhitzt. Der nach Destillation erhaltene Destillationsrückstand wiegt 442 g und enthält 1,945 Mol (412 g) 3-Benzoylaminoanilin.

## Patentansprüche

1. Verfahren zur Herstellung von 3-Acylaminoanilinen der allgemeinen Formel (1)

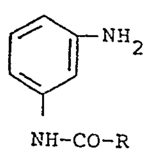

(1)

in welcher R einen verzweigten oder unverzweigten Alkylrest mit 1-7 Kohlenstoffatomen oder die Phenylgruppe, die durch 1-2 Methylgruppen oder durch 1-2 Chloratome substituiert sein kann, bedeutet, dadurch gekennzeichnet, dass man eine Carbonsäure der allgemeinen Formel (2)

$$R-COOH \qquad (2)$$

in welcher R die genannte Bedeutung hat, mit einem Überschuss an 1,3-Diaminobenzol bei 120 bis 240° C umsetzt und die Reaktionsmischung durch Destillation trennt.

2. Verfahren nach Anspruch 1, dadurch gekennzeichnet, dass man das 1,3-Diaminobenzol in einem zwei- bis zehnfachen molaren Überschuss einsetzt.

3. Verfahren nach Ansprüchen 1 oder 2, dadurch gekennzeichnet, dass man das bei der Reaktion entstehende Wasser während der Reaktion abdestillieren lässt.

4. Verfahren nach Ansprüchen 1 bis 3, dadurch gekennzeichnet, dass man die an die Reaktion anschliessende Destillation bei vermindertem Druck von 0,1 bis 50 Torr durchführt.

## Claims

1. A process for the preparation of 3-acylaminoanilines of the general formula (1)

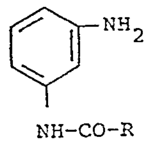

(1)

in which R denotes a branched or unbranched alkyl radical having 1-7 carbon atoms or the phenyl group, which can be substituted by 1-2 methyl groups or by 1-2 chlorine atoms, which comprises reacting a carboxylic acid of the general formula (2)

$$R-COOH \qquad (2)$$

in which R has the meaning mentioned, with an excess of 1,3-diaminobenzene at 120 to 240°C, and separating the reaction mixture by distillation.

2. The process as claimed in claim 1, wherein the 1,3-diaminobenzene is employed in a two-to ten-fold molar excess.

3. The process as claimed in claims 1 or 2, wherein the water being produced in the reaction is allowed to distil out during the reaction.

4. The process as claimed in any of claims 1 to 3, wherein distillation under reduced pressure of 0.1 to 50 torr is carried out after the reaction.

## Revendications

1. Procédé de préparation d'acylamino-3 anilines répondant à la formule générale (1):

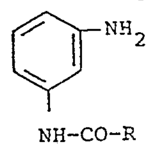

(1)

dans laquelle R représente un radical alkyle linéaire ou ramifié qui contient de 1 à 7 atomes de carbone ou représente un radical phényle qui porte éventuellement 1 ou 2 radicaux méthyles ou 1 ou 2 atomes de chlore, procédé caractérisé en ce qu'on fait réagir un acide carboxylique répondant à la formule générale (2):

$$R-COOH \qquad (2)$$

dans laquelle R a la signification précédemment donnée, avec un excès de diamino-1,3 benzène, à une température de 120 à 240° C, et on sépare le mélange réactionnel par distillation.

2. Procédé selon la revendication 1, caractérisé en ce qu'on utilise le diamino-1,3 benzène en un excès molaire de 2 à 10 fois.

3. Procédé selon l'une des revendications 1 et 2, caractérisé en ce que l'eau engendrée lors de la réaction est chassée au cours de celle-ci par distillation.

4. Procédé selon l'une quelconque des revendications 1 à 3, caractérisé en ce que la distillation qui fait suite à la réaction est exécutée sous pression réduite, en l'espèce entre 0,1 et 50 torr.